# EUROPEAN PATENT APPLICATION

(11) **EP 3 747 367 A2**
(43) Date of publication of application: **09.12.2020**
(21) Application number: 20175720.0
(22) Date of filing: 20.05.2020
(51) Int. Cl.: A61B 5/16, G06F 17/18, G06F 3/01, G06N 20/00

(54) **INFORMATION PROCESSING SYSTEM**

(30) Priority: 27.05.2019 JP 2019098579
(71) Applicant: JTEKT CORPORATION, Chuo-ku, Osaka-shi, Osaka 542-8502 (JP)
(72) Inventor: SHIMIZU, Yohei, Osaka-shi, Osaka 542-8502 (JP)
(74) Representative: Winter, Brandl, Fürniss, Hübner, Röss, Kaiser, Polte - Partnerschaft mbB

(57) **Abstract**

An information processing system is configured to generate data for identifying an emotion of a person touching a deformable structure (11). The information processing system includes a sensor provided in the structure (11) and configured to output a signal indicating a temporal change of a physical quantity in the structure (11) due to a touch by the person; and an electronic control unit (30) configured to i) perform fast Fourier transform on the signal at a sampling frequency that is equal to or less than 10 Hz to generate frequency domain data, ii) quantize data with a frequency equal to or less than half the sampling frequency among the frequency domain data into a predetermined number of frequency bands to generate emotion identification data, and iii) perform machine learning using the emotion identification data as input data, to generate classification boundary data for classifying data serving as a processing target according to emotion categories.

## Description

### BACKGROUND OF THE INVENTION

### 1. Field of the Invention

The invention relates to an information processing system that generates data used to identify a person's emotion, and an information processing system that identifies a person's emotion.

### 2. Description of Related Art

In the field of robotics, or in a new mobility society involving Mobility as a Service (MaaS) and a flying car, emotions and intentions of a person (user) are recognized, and a state of a structural component such as an abnormality is detected. Accordingly, a larger amount of information than what is currently available is required.

In such a technique described above, a polymer material (rubber, resin, or elastomer) is used as exterior materials, interior materials, or drive parts, for example. To obtain information on a person's emotion, a soft material sensor is attached to a surface of an interior material made of, for example, elastomer. The soft material sensor is deformed in accordance with deformation of the interior material, and outputs a signal based on the deformation. Japanese Unexamined Patent Application Publication No. 2013-178241 (JP 2013-178241 A) discloses a technique in which pressure sensing sensors are provided on a flexible printed circuit board.

### SUMMARY OF THE INVENTION

As described above, the soft material sensor is attached to the surface of the interior material to obtain information on a person's emotion.

Even when a person touches the interior material and the soft material sensor responds and outputs a signal, it is unknown what the signal means, and the person's emotion when the person touches the interior material cannot be identified.

The present disclosure provides an information processing system that generates data for identifying a person's emotion when the person touches a deformable structure, and an information processing system for identifying the person's emotion when the person touches a deformable structure.

A first aspect of the disclosure relates to an information processing system configured to generate data for identifying an emotion of a person who touches a structure that is deformable. The information processing system includes a sensor provided in the structure and configured to output a signal indicating a temporal change of a physical quantity in the structure due to a touch by the person; and an electronic control unit configured to i) perform fast Fourier transform on the signal at a sampling frequency that is equal to or less than 10 Hz to generate frequency domain data, ii) quantize data with a frequency equal to or less than half the sampling frequency among the frequency domain data into a predetermined number of frequency bands to generate emotion identification data, and iii) perform machine learning using the emotion identification data as input data, to generate classification boundary data for classifying data serving as a processing target according to emotion categories.

With the information processing system, the classification boundary data for classifying data serving as a processing target according to emotion categories is generated based on the signal obtained by the sensor when the person touches the structure. Based on the generated classification boundary data, the person's emotion when the person touches the structure can be identified.

The sensor may include a plurality of sensors provided in the structure; each of the sensors may be configured to output the signal indicating the temporal change of the physical quantity in the structure; and the emotion identification data may include data based on the signals output from the sensors, and data representing a difference between the data based on the signals output from the sensors. In this case, even when the structure is deformed in a complicated manner, it is possible to obtain the classification boundary data (learned model) that makes it possible to identify an emotion with high accuracy.

The electronic control unit may be configured to i) perform the fast Fourier transform on the signal at a first sampling frequency that is equal to or less than 10 Hz to generate first frequency domain data that is the frequency domain data, ii) perform the fast Fourier transform on the signal at a second sampling frequency that is greater than 10 Hz to generate second frequency domain data, iii) quantize data with a frequency greater than half the second sampling frequency among the second frequency domain data into a predetermined number of frequency bands to generate abnormality identification data, and iv) perform machine learning using the abnormality identification data as input data, to generate identification boundary data for distinguishing an abnormality of the sensor from a normal state of the sensor. In this case, it is possible to obtain the identification boundary data that makes it possible to perform a failure diagnosis for a mechanism including the sensor and the structure for identifying an emotion.

A second aspect of the disclosure relates to an information processing system configured to identify an emotion of a person who touches a structure that is deformable. The information processing system includes a sensor provided in the structure and configured to output a signal indicating a temporal change of a physical quantity in the structure due to a touch by the person; and an electronic control unit including a storage unit configured to store classification boundary data for classifying data serving as a processing target according to emotion categories. The electronic control unit is configured to i) perform preprocessing using the signal from the sensor as actual data to generate target input data, ii) determine which emotion category the target input data belongs to, based on the classification boundary data, iii) to perform fast Fourier transform on the signal that is the actual data at a sampling frequency that is equal to or less than 10 Hz to generate frequency domain data, and iv) quantize data with a frequency equal to or less than half the sampling frequency among the frequency domain data into a predetermined number of frequency bands to generate the target input data.

With the information processing system, the person's emotion when the person touches the structure can be classified according to categories, based on the signal obtained by the sensor when the person touches the structure. That is, it is possible to identify the person's emotion when the person touches the structure.

The electronic control unit may be configured to i) perform the fast Fourier transform on the signal at the sampling frequency that is equal to or less than 10 Hz to generate the frequency domain data, ii) quantize the data with the frequency equal to or less than half the sampling frequency among the frequency domain data into the predetermined number of frequency bands to generate emotion identification data, iii) perform machine learning using the emotion identification data as input data, to generate the classification boundary data, and iv) store the classification boundary data in the storage unit.

The sensor may include a plurality of sensors provided in the structure; each of the sensors may be configured to output the signal indicating the temporal change of the physical quantity in the structure; and the target input data may include data based on the signals output from the sensors, and data representing a difference between the data based on the signals output from the sensors. In this case, even when the structure is deformed in a complicated manner, it is possible to increase the accuracy in classifying the data serving as a processing target according to emotion categories

The storage unit may be configured to store identification boundary data for distinguishing an abnormality of the sensor from a normal state of the sensor; the electronic control unit may be configured to perform the fast Fourier transform on the signal that is the actual data at a first sampling frequency that is equal to or less than 10 Hz to generate first frequency domain data that is the frequency domain data, and perform the fast Fourier transform on the signal that is the actual data at a second sampling frequency that is greater than 10 Hz to generate second frequency domain data; and the electronic control unit may be configured to quantize data with a frequency greater than half the second sampling frequency among the second frequency domain data into a predetermined number of frequency bands to generate second target input data, and determine whether the second target input data is data corresponding to an abnormality or data corresponding to a normal state, based on the identification boundary data. In this case, the information processing system can perform a failure diagnosis for the sensor by itself without using another mechanism.

The electronic control unit may be configured to i) perform the fast Fourier transform on the signal at the second sampling frequency that is greater than 10 Hz to generate the second frequency domain data, ii) quantize the data with the frequency greater than half the second sampling frequency among the second frequency domain data into the predetermined number of frequency bands to generate abnormality identification data, iii) perform machine learning using the abnormality identification data as input data, to generate the identification boundary data, and iv) to store the identification boundary data in the storage unit.

With the invention according to the above aspects of the present disclosure, it is possible to identify a person's emotion when the person touches the structure that is deformable.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
FIG. 1 is an explanatory diagram schematically showing a configuration of an information processing system;
FIG. 2 is a flowchart showing processes of generating classification boundary data;
FIG. 3 includes graphs showing examples of a signal from a sensor;
FIG. 4 is a graph showing an example of first frequency region data;
FIG. 5 is a graph showing an example of the classification boundary data; and
FIG. 6 is a flowchart showing processes of identifying a person's emotion.

### DETAILED DESCRIPTION OF EMBODIMENTS

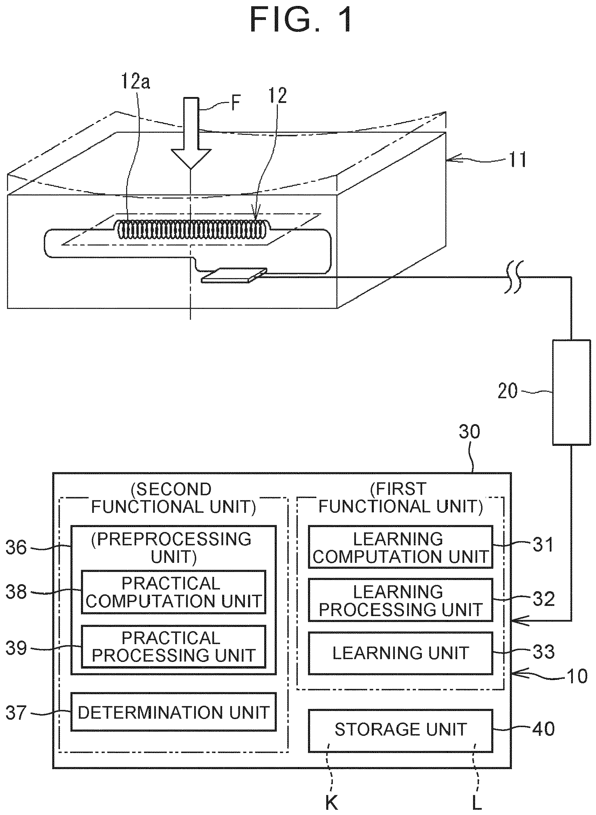

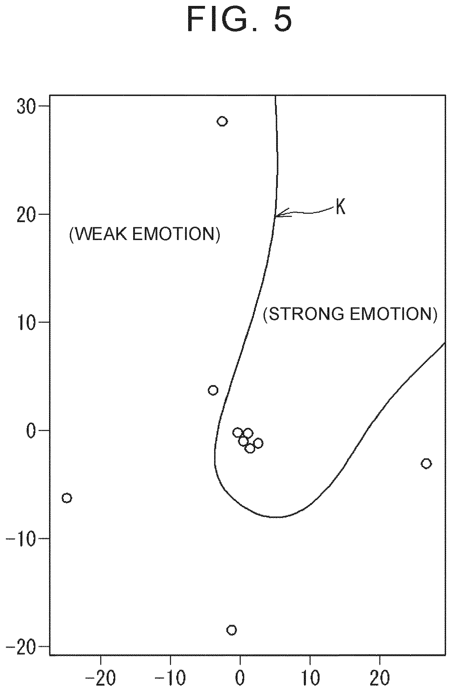

FIG. 1 is an explanatory diagram schematically showing a configuration of an information processing system. An information processing system 10 of the present disclosure is configured to identify an emotion of a person who has touched a deformable structure 11. The information processing system 10 can generate classification boundary data used for identifying the emotion of the person who has touched the structure 11. The classification boundary data is information for classifying input data (data serving as a processing target) according to categories of a person's emotion, and the classification boundary data is generated based on a learned model by machine learning. The learned model is classification boundary data K (see FIG. 5) described later, and is generated by the information processing system 10 of the present disclosure. Emotion categories include, for example, calmness, sadness, impatience, and anger.

Further, the information processing system 10 of the present disclosure can also detect an abnormality of a sensor 12 provided in the structure 11. For abnormality detection, the information processing system 10 generates identification boundary data for distinguishing the abnormality of the sensor 12 from a normal state of the sensor 12. The function of generating the identification boundary data and the function of detecting the abnormality may be omitted.

The information processing system 10 includes a sensor 12 provided in the structure 11, and a computation processing device 30 that processes a signal output from the sensor 12. The computation processing device 30 is a computer including a processor (central processing unit (CPU)), a storage device, an input/output device, and the like. In other words, an example of the computation processing device 30 is an electronic control unit (ECU). Each function of the computation processing device 30 is exhibited (implemented) when the processor executes a computer program stored in the computer. Each function of the computation processing device 30 will be described later.

The structure 11 is made of a polymer material and can be elastically deformed by an external force F when a person touches the structure 11. In response to the deformation of the structure 11 due to the external force F, the sensor 12 outputs a signal. The structure 11 of the present disclosure is made of elastomer. Alternatively, the structure 11 may be made of rubber. The structure 11 is applied to, for example, an interior material such as a steering wheel, an armrest, or a seat of a vehicle (e.g., a vehicle). When the structure 11 is applied to an interior material such as an armrest or a seat, the structure 11 is a part of the interior material and has a prismatic shape (a prismatic block shape) as shown in FIG. 1. When the structure 11 is applied to a part of a steering wheel of a vehicle, the structure 11 has a columnar shape. The shape of the structure 11 may be a shape other than the prismatic shape and the columnar shape and may vary depending on an object to which the structure 11 is applied.

The sensor 12 is provided in the structure 11 so as to be embedded in the structure 11. The sensor 12 is deformed together with the structure 11 by the external force F. The sensor 12 of the present disclosure includes a metal fiber coil 12a. An alternating current (AC) signal is input to the coil 12a. When the structure 11 is deformed by receiving the external force F, the coil 12a is also deformed in accordance with the deformation of the structure 11. Due to this deformation, an inductance of the coil 12a changes. This change in the inductance causes a change in the signal output from the sensor 12.

The sensor 12 includes the coil 12a and an LCR meter 20 serving as a detection unit. The LCR meter 20 detects the inductance of the coil 12a. The LCR meter 20 outputs the detected inductance as a signal to the computation processing device 30. The computation processing device 30 obtains a change (a temporal change i.e., a change with respect to time) in the inductance based on the signal. That is, the sensor 12 having the above-described configuration outputs a signal (that is, a waveform signal) indicating a temporal change in the deformation of the structure 11 due to a touch by a person.

The signal output from the sensor 12 may be a signal other than a waveform signal indicating the deformation of the structure 11. For example, the output signal from the sensor 12 may be a waveform signal indicating a force acting on the structure 11. The output signal from the sensor 12 may be a waveform signal indicating a physical quantity in the structure 11 generated by a touch by a person. Hereinafter, a case in which the physical quantity is deformation (displacement) will be described.

The sensor 12 may have other configurations. For example, the sensor 12 may be a sensor including a film-shape dielectric elastomer and a pair of film-shaped elastic electrodes. The dielectric elastomer is interposed between the pair of electrodes. When the structure 11 is deformed by receiving the external force, the dielectric elastomer and the electrodes (sensor) are deformed in accordance with the deformation of the structure 11. Due to this deformation, a capacitance of the sensor changes. This change in the capacitance causes a change in the output signal from the sensor. Alternatively, the sensor 12 may include an ionic polymer-metal composite (IPMC) or a fluorine-based piezoelectric element.

A plurality of sensors 12 may be provided in the structure 11. For example, when the structure 11 is applied to a part of a steering wheel of a vehicle, a person touches (grips) a part of the steering wheel (the structure 11) with his/her hand such that the part of the steering wheel is gripped by the hand from two directions. Thus, in the structure 11, each of the sensors 12 is provided at two positions that the person is likely to touch from two directions. Note that the number of sensors 12 is not limited to two, and may be three or more. Each of the sensors 12 outputs a signal (that is, a waveform signal) indicating a temporal change in the deformation of the structure 11. The computation processing device 30 can obtain the signal of each of the sensors 12 separately, and process the signal of each of the sensors 12 separately.

The computation processing device 30 includes a learning computation unit 31, a learning processing unit 32, and a learning unit 33 as a first functional unit. The functions of the first functional unit are achieved by a processor executing a computer program. The computation processing device 30 includes a preprocessing unit 36 and a determination unit 37 as a second functional unit. The functions of the second functional unit are achieved by a processor executing a computer program. The preprocessing unit 36 includes a practical computation unit 38 and a practical processing unit 39.

The first functional unit includes functions for generating classification boundary data K (see FIG. 5) for classifying data serving as a processing target according to emotion categories. FIG. 5 is a conceptual graph showing the classification boundary data K. The second functional unit has functions for identifying a person's emotion from data obtained when the person (user) actually touches the structure 11 by using the classification boundary data K generated by the first functional unit.

The computation processing device 30 includes a storage unit 40. The storage unit 40 includes a nonvolatile memory element such as a flash memory or a magnetic storage device such as a hard disk. The storage unit 40 stores the output signal from the sensor 12, data obtained by processing the output signal, the classification boundary data K, identification boundary data described later, and the like.

Hereinafter, the sensor 12 and each functional unit of the computation processing device 30 and the processes performed by the information processing system 10 will be described. First, generation of the classification boundary data K (and the identification boundary data) by machine learning will be described. Thereafter, processes of identifying a person's emotion (and detecting an abnormality of the sensor 12) based on the generated classification boundary data K (and the identification boundary data) will be described.

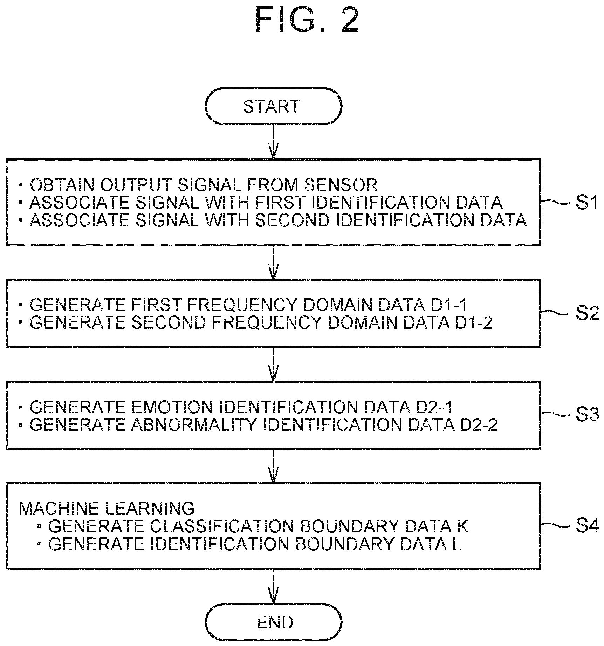

FIG. 2 is a flowchart showing processes of generating the classification boundary data K (and the identification boundary data).

The AC signal is input to the sensor 12 that includes the coil 12a. A high-frequency wave (100 kHz to 500 kHz) is applied to the coil 12a, and a change in the inductance is obtained by the computation processing device 30. Specifically, the value of the inductance is measured at a measurement frequency of 100 kHz to 500 kHz, and this measurement is performed every 0.1 seconds. This measurement is repeatedly performed for a predetermined time. The predetermined time corresponds to one processing cycle. The processing cycle is repeatedly performed. The predetermined time is, for example, 20 seconds. Since the measurement is performed every 0.1 seconds, 200 pieces of measurement data on inductances can be obtained in one processing cycle.

When a person touches the structure 11, an external force F is applied to the structure 11. Processes performed later include supervised machine learning. A person touches the structure 11 with various emotions and deforms the structure 11. Types (kinds) of the emotions include, for example, calmness, sadness, impatience, and anger. In the following, for ease of description, the types of the emotions are classified into two emotions, namely a "strong emotion" and a "weak emotion". A strong emotion is, for example, anger, and a weak emotion is, for example, calmness. The measurement data is obtained for each emotion. Emotion data is linked to (associated with) the measurement data and stored in the storage unit 40.

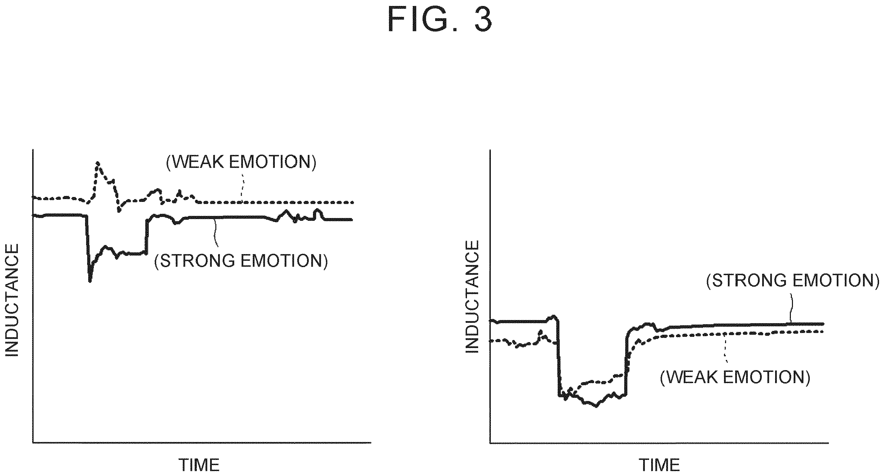

FIG. 3 includes graphs showing examples of an output signal from the sensor 12. The output signal from the sensor 12 in the case of a strong emotion is different from the output signal from the sensor 12 in the case of a weak emotion. FIG. 3 shows two graphs. In this case, two sensors 12 are provided in the structure 11, and the signals output from the two sensors 12 are shown in FIG. 3.

As described above, the sensor 12 outputs a signal indicating a temporal change in the deformation of the structure 11 due to a touch by a person, and the computation processing device 30 obtains the signal as measurement data for each emotion (step S1 in FIG. 2). The output signal (measurement data) from the sensor 12 is associated with first identification data indicating whether the data represents a strong emotion or a weak emotion.

In step S1, a person touches the structure 11 in a state in which noise is applied to the sensor 12 provided in the structure 11 and in a normal state in which no noise is applied to the sensor 12. The output signal from the sensor 12 is associated with second identification data indicating whether the sensor 12 is in a state in which noise is applied or a normal state in which noise is not applied.

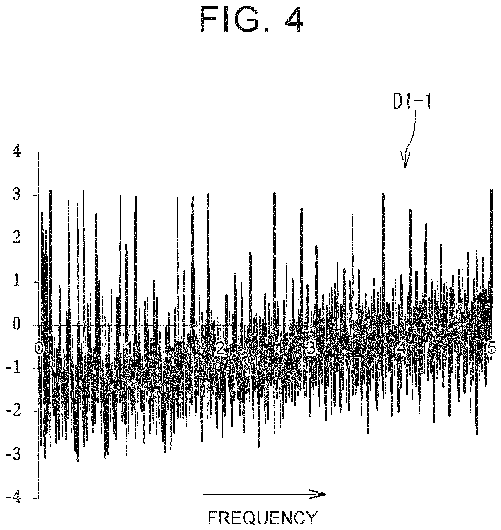

The learning computation unit 31 performs fast Fourier transform on each measurement data based on the signal transmitted from the sensor 12 to the computation processing device 30, at a sampling frequency that is equal to or less than 10 Hz, thereby generating first frequency domain data D1-1 (step S2 in FIG. 2). FIG. 4 is a graph showing an example of the first frequency domain data D1-1.

The learning computation unit 31 has a function of performing fast Fourier transform at two sampling frequencies. That is, as described above, the learning computation unit 31 performs fast Fourier transform on the signal from the sensor 12 at a first sampling frequency that is equal to or less than 10 Hz, thereby generating the first frequency domain data D1-1 (step S2 in FIG. 2). Further, the learning computation unit 31 performs fast Fourier transform on the signal from the sensor 12 at a second sampling frequency that is greater than 10 Hz, thereby generating second frequency domain data D1-2 (step S2 in FIG. 2). In the present disclosure, the first sampling frequency is 10 Hz. The second sampling frequency is, for example, 100 Hz to 1000 Hz, and is 100 Hz in the present disclosure.

The learning processing unit 32 quantizes data with a frequency equal to or less than half the first sampling frequency (5 Hz in the present disclosure) among the first frequency domain data D1-1 into a predetermined number of frequency bands. Thus, emotion identification data D2-1 is generated (step S3 in FIG. 2). In the present disclosure, the "predetermined number" is "512". Further, the learning processing unit 32 quantizes data with a frequency greater than half the second sampling frequency (50 Hz in the present disclosure) among the second frequency domain data D1-2 into a predetermined number of frequency bands. Thereby, abnormality identification data D2-2 is generated (step S3 in FIG. 2). In the present disclosure, the "predetermined number" is "512".

The learning unit 33 performs machine learning using the emotion identification data D2-1 as input data (explanatory variables). The machine learning used here is supervised machine learning. In particular, machine learning using a support vector machine, which is one of the pattern recognition models using supervised learning, is performed. The learning unit 33 generates the classification boundary data K for classifying the data serving as a processing target according to emotion categories by machine learning (step S4 in FIG. 2). FIG. 5 is a graph showing an example of the classification boundary data K. In FIG. 5, the classification boundary data K is indicated by a curve on two-dimensional coordinates.

For supervised learning, as described above, in step S1, the first identification data indicating a person's emotion when the person touches the structure 11 is associated with a signal obtained from the sensor 12. Thus, the first identification data is also associated with the emotion identification data D2-1 serving as input data. This makes it possible to perform supervised machine learning. By this machine learning, the classification boundary data K for classifying the data serving as a processing target according to emotion categories is generated (step S4 in FIG. 2). An example of the classification boundary data K is indicated by a solid line in FIG. 5.

Further, the learning unit 33 performs machine learning using the abnormality identification data D2-2 as input data (explanatory variables). The machine learning used here is supervised machine learning. As with the case of the classification boundary data K, machine learning using a support vector machine is performed. The learning unit 33 generates identification boundary data L for distinguishing the abnormality of the sensor 12 from the normal state of the sensor 12 by machine learning.

For supervised learning, as described above, in step S1, a person touches the structure 11 in a state in which noise is applied to the sensor 12 provided in the structure 11 and in a normal state in which no noise is applied. The signal obtained from the sensor 12 and data indicating the abnormality or the normal state of the sensor 12 are linked to each other. That is, the signal from the sensor 12 is associated with the second identification data indicating whether the person touches the structure 11 in the state in which noise is applied to the sensor 12 or in the normal state in which noise is not applied to the sensor 12. Thus, the second identification data is also associated with the abnormality identification data D2-2 serving as input data. This makes it possible to perform supervised machine learning. With this machine learning, the identification boundary data L for identifying (classifying) the data serving as a processing target as the data indicating the abnormality of the sensor 12 or the data indicating the normal state of the sensor 12 is generated (step S4 in FIG. 2).

In the present disclosure, the structure 11 is provided with two sensors 12. Each of the two sensors 12 outputs a signal (waveform signal) indicating a temporal change in the deformation of the structure 11, and the computation processing device 30 obtains each signal in step S1 in FIG. 2. The learning computation unit 31 performs fast Fourier transform on each of the signals described above. As a result, in step S2 in FIG. 2, the first frequency domain data D1-1 (A) and the second frequency domain data D1-2 (A) based on the output signal from a first sensor 12 are generated. Further, first frequency domain data D1-1 (B) and second frequency domain data D1-2 (B) based on the output signal from a second sensor 12 are generated.

In step S3 in FIG. 2, the learning processing unit 32 performs quantization processes on each of the first frequency domain data D1-1 (A), D1-1 (B) and the second frequency domain data D1-2 (A), D1-2 (B) based on the two sensors 12. As a result, emotion identification data D2-1 (A), D2-1 (B) and abnormality identification data D2-2 (A), D2-2 (B) based on the two sensors 12 are generated.

The learning unit 33 performs machine learning as described above. Data used as the explanatory variables in the machine learning include, in addition to the emotion identification data D2-1 (A) based on the output signal from the first sensor 12 and the emotion identification data D2-1 (B) based on the output signal from the second sensor 12, data [D2-1 (A) - D2-1 (B)] representing the difference between the data D2-1 (A) and the data D2-1 (B). That is, the emotion identification data D2-1 used in the machine learning include data representing the difference between the data based on the signals output from the two sensors 12, in addition to the data based on the signals output from the two sensors 12. In this case, even when the structure 11 is deformed in a complicated manner, the classification boundary data K (learned model) with highly accurate emotion identification is obtained.

The learning unit 33 generates the identification boundary data L for abnormality detection. Also in this case, the abnormality identification data D2-2 used in the machine learning include, in addition to the data D2-2 (A), D2-2 (B) based on the signals output from the two sensors 12, data [D2-2 (A) - D2-2 (B)] representing the difference between the data based on the signals output from the two sensors 12.

As described above, the classification boundary data K and the identification boundary data L are generated by the learning computation unit 31, the learning processing unit 32, and the learning unit 33. The classification boundary data K and the identification boundary data L are stored in the storage unit 40. Using the classification boundary data K and the identification boundary data L, the computation processing device 30 can identify the emotion of a person who actually touches the structure 11, and can also perform abnormality detection. In other words, generation of the classification boundary data K and the identification boundary data L allows the information processing system 10 to perform actual operation for identifying a person's emotion and performing abnormality detection. The functions of the information processing system 10 used in the actual operation and the processes of the operation will be described below.

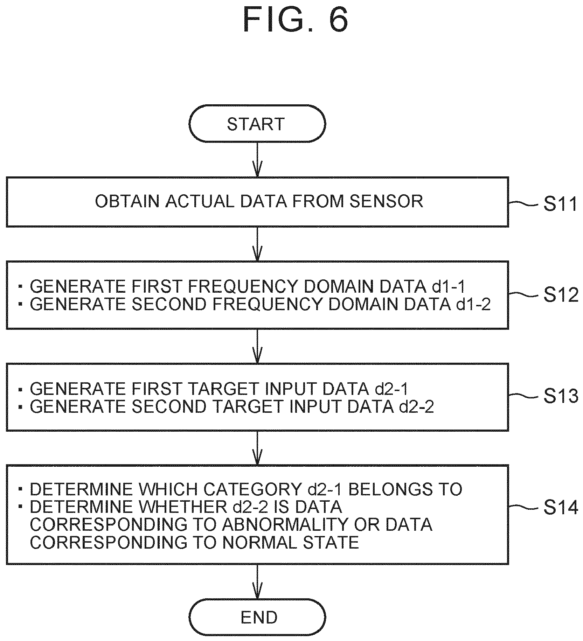

Identification of a person's emotion based on the generated classification boundary data K will be described. FIG. 6 is a flowchart showing processes of identifying a person's emotion. When a person touches the structure 11 with a certain emotion, the structure 11 is deformed by the external force F. Then, a signal is output from the sensor 12. This signal is referred to as "actual data". The computation processing device 30 obtains the actual data (step S11 in FIG. 6). The state of the sensor 12 in step S11 is the same as that in step S1 shown in FIG. 2. A high-frequency wave (100 kHz to 500 kHz) is applied to the sensor 12, and the value of the inductance is measured. This value is a signal output from the sensor 12, and the computation processing device 30 obtains this signal as the actual data.

The preprocessing unit 36 has a function of performing preprocessing using the signal from the sensor 12, that is, the actual data. By performing the preprocessing, target input data is generated from the signal (actual data) from the sensor 12. To perform the preprocessing, the preprocessing unit 36 includes the practical computation unit 38 and the practical processing unit 39.

The practical computation unit 38 performs fast Fourier transform on a signal from the sensor 12 that is the actual data at a first sampling frequency that is equal to or less than 10 Hz, thereby generating first frequency domain data d1-1 (step S12 in FIG. 6). Further, the practical computation unit 38 performs fast Fourier transform on a signal from the sensor 12 that is the actual data at a second sampling frequency that is greater than 10 Hz, thereby generating second frequency domain data d1-2 (step S12 in FIG. 6). The function of the practical computation unit 38 is the same as that of the learning computation unit 31 described with regard to the first functional unit. Therefore, the practical computation unit 38 can substitute for the learning computation unit 31, and the learning computation unit 31 can substitute for the practical computation unit 38.

The practical processing unit 39 quantizes data with a frequency equal to or less than half the first sampling frequency among the first frequency domain data d1-1 into a predetermined number of frequency bands, thereby generating first target input data d2-1 (step S13 in FIG. 6). The practical processing unit 39 quantizes data with a frequency greater than half the second sampling frequency among the second frequency domain data d1-2 into a predetermined number of frequency bands, thereby generating second target input data d2-2 (step S13 in FIG. 6). In the present disclosure, the "predetermined number" is "512", which is the same as the "predetermined number" in the case of the learning processing unit 32 described with regard to the first functional unit. The function of the practical processing unit 39 is the same as that of the learning processing unit 32 described with regard to the first functional unit. Therefore, the practical processing unit 39 can substitute for the learning processing unit 32, and the learning processing unit 32 can substitute for the practical processing unit 39.

The determination unit 37 determines which emotion category the first target input data d2-1 belongs to based on the classification boundary data K stored in the storage unit 40 (step S14 in FIG. 6). For this purpose, the generated first target input data d2-1 is used as an input to a learned model indicating the classification boundary data K. Thus, the determination unit 37 can obtain an output (a person's emotion category) corresponding to an input (first target input data d2-1) to the learned model (classification boundary data K). That is, based on the first target input data d2-1, an estimation is made on a person's emotion when a signal that is the basis of the first target input data d2-1 is obtained from the sensor 12.

The determination unit 37 can determine whether the second target input data d2-2 is data corresponding to an abnormality or data corresponding to a normal state, based on the identification boundary data L stored in the storage unit 40 (step S14 in FIG.

6). For this purpose, the generated second target input data d2-2 is used as an input to a learned model indicating the identification boundary data L. Thus, the determination unit 37 can obtain an output (abnormality or normal state) corresponding to an input (second target input data d2-2) to the learned model (identification boundary data L). That is, based on the second target input data d2-2, an estimation is made (i.e., a determination is made) on whether an abnormality has occurred in the sensor 12 or the sensor 12 is in a normal state when a signal that is the basis of the second target input data d2-2 is obtained from the sensor 12.

In the present disclosure, the structure 11 is provided with two sensors 12. Thus, when the classification boundary data K is used to identify (estimate) a person's emotion, data used as input data to be input to the classification boundary data K include, in addition to the target input data d2-1 (A), d2-1 (B) based on the signals output from the two first and second sensors 12, data [d2-1 (A) - d2-1 (B)] representing the difference between the first target input data d2-1 (A) based on the output signal from the first sensor 12 and the second target input data d2-1 (B) based on the output signal from the second sensor 12. In this case, even when the structure 11 is deformed in a complicated manner, it is possible to increase the accuracy in classifying the actual data serving as a processing target according to emotion categories.

Similarly, data used as input data to be input to the identification boundary data L for abnormality detection include data [d2-1 (A) - d2-1 (B)] representing the difference between the first target input data d2-1 (A) based on the output signal from the first sensor 12 and the second target input data d2-1 (B) based on the output signal from the second sensor 12.

As described above, the information processing system 10 of the present disclosure has a function of generating data for identifying an emotion of a person who has touched the deformable structure 11. The data that is generated is the classification boundary data K, which is the data of a learned model for classifying data serving as a processing target (actual data) according to emotion categories. For this purpose, the information processing system 10 includes the sensor 12 and the computation processing device 30. The sensor 12 is provided in the structure 11 and outputs a signal indicating a temporal change of the deformation of the structure 11 due to a touch by a person. The computation processing device 30 includes the learning computation unit 31, the learning processing unit 32, and the learning unit 33.

The learning computation unit 31 performs fast Fourier transform on the signal from the sensor 12 at a sampling frequency that is equal to or less than 10 Hz, thereby generating the first frequency domain data D1-1 (step S2 in FIG. 2). The learning processing unit 32 quantizes data with a frequency equal to or less than half the first sampling frequency among the first frequency domain data D1-1 into a predetermined number of frequency bands, thereby generating the emotion identification data D2-1 (step S3 in FIG. 2). The learning unit 33 performs machine learning using the emotion identification data D2-1 as the input data to generate the classification boundary data K as the data of the learned model (step S4 in FIG. 2).

With the information processing system 10, the classification boundary data K is generated based on a signal obtained by the sensor 12 when a person touches the structure 11. Generation of the classification boundary data K allows the information processing system 10 to identify, based on the classification boundary data K, a person's emotion when the person touches the structure 11.

Further, the information processing system 10 of the present disclosure identifies, based on the classification boundary data K, an emotion of a person who has touched the structure 11. For this purpose, the information processing system 10 includes the storage unit 40 including a hard disk or the like. The classification boundary data K is stored in the storage unit 40. The classification boundary data K stored in the storage unit 40 is data of a learned model (classification boundary data K) generated by the learning unit 33.

The information processing system 10 includes the preprocessing unit 36 and the determination unit 37. The preprocessing unit 36 performs preprocessing using a signal from the sensor 12 as actual data to generate the target input data d2-1. The preprocessing is performed as follows. The preprocessing unit 36 (practical computation unit 38) performs fast Fourier transform on the signal that is the actual data at a sampling frequency that is equal to or less than 10 Hz, thereby generating the frequency domain data d1-1 (step S12 in FIG. 6). The preprocessing unit 36 (practical processing unit 39) quantizes data with a frequency equal to or less than half the first sampling frequency among the first frequency domain data d1-1 into a predetermined number of frequency bands, thereby generating the first target input data d2-1 (step S13 in FIG. 6). The determination unit 37 determines which emotion category the target input data d2-1 belongs to based on the classification boundary data K (step S14 in FIG. 6).

With the information processing system 10, it is possible to classify a person's emotion when the person touches the structure 11 according to categories, based on a signal obtained by the sensor 12 when the person touches the structure 11. In other words, it is possible to identify the person's emotion when the person touches the structure 11.

The embodiments in the disclosure are illustrative and not restrictive in all respects. The scope of the invention is not limited to the above-described embodiments, but includes all modifications within the scope equivalent to the configuration described in the claims.

## Claims

1. An information processing system configured to generate data for identifying an emotion of a person who touches a structure (11) that is deformable, the information processing system **characterized by** comprising:
a sensor provided in the structure (11) and configured to output a signal indicating a temporal change of a physical quantity in the structure (11) due to a touch by the person; and
an electronic control unit (30) configured to i) perform fast Fourier transform on the signal at a sampling frequency that is equal to or less than 10 Hz to generate frequency domain data, ii) quantize data with a frequency equal to or less than half the sampling frequency among the frequency domain data into a predetermined number of frequency bands to generate emotion identification data, and iii) perform machine learning using the emotion identification data as input data, to generate classification boundary data for classifying data serving as a processing target according to emotion categories.

2. The information processing system according to claim 1, **characterized in that**:
the sensor includes a plurality of sensors provided in the structure (11);
each of the sensors is configured to output the signal indicating the temporal change of the physical quantity in the structure (11); and
the emotion identification data includes data based on the signals output from the sensors, and data representing a difference between the data based on the signals output from the sensors.

3. The information processing system according to claim 1 or 2, **characterized in that** the electronic control unit (30) is configured to i) perform the fast Fourier transform on the signal at a first sampling frequency that is equal to or less than 10 Hz to generate first frequency domain data that is the frequency domain data, ii) perform the fast Fourier transform on the signal at a second sampling frequency that is greater than 10 Hz to generate second frequency domain data, iii) quantize data with a frequency greater than half the second sampling frequency among the second frequency domain data into a predetermined number of frequency bands to generate abnormality identification data, and iv) perform machine learning using the abnormality identification data as input data, to generate identification boundary data for distinguishing an abnormality of the sensor from a normal state of the sensor.

4. An information processing system configured to identify an emotion of a person who touches a structure (11) that is deformable, the information processing system **characterized by** comprising:
a sensor provided in the structure (11) and configured to output a signal indicating a temporal change of a physical quantity in the structure (11) due to a touch by the person; and
an electronic control unit (30) including a storage unit (40) configured to store classification boundary data for classifying data serving as a processing target according to emotion categories, the electronic control unit (30) being configured to i) perform preprocessing using the signal from the sensor as actual data to generate target input data, ii) determine which emotion category the target input data belongs to, based on the classification boundary data, iii) to perform fast Fourier transform on the signal that is the actual data at a sampling frequency that is equal to or less than 10 Hz to generate frequency domain data, and iv) quantize data with a frequency equal to or less than half the sampling frequency among the frequency domain data into a predetermined number of frequency bands to generate the target input data.

5. The information processing system according to claim 4, **characterized in that** the electronic control unit (30) is configured to i) perform the fast Fourier transform on the signal at the sampling frequency that is equal to or less than 10 Hz to generate the frequency domain data, ii) quantize the data with the frequency equal to or less than half the sampling frequency among the frequency domain data into the predetermined number of frequency bands to generate emotion identification data, iii) perform machine learning using the emotion identification data as input data, to generate the classification boundary data, and iv) store the classification boundary data in the storage unit (40).

6. The information processing system according to claim 4 or 5, **characterized in that**:
the sensor includes a plurality of sensors provided in the structure (11);
each of the sensors is configured to output the signal indicating the temporal change of the physical quantity in the structure (11); and
the target input data includes data based on the signals output from the sensors, and data representing a difference between the data based on the signals output from the sensors.

7. The information processing system according to any one of claims 4 to 6, **characterized in that**:
the storage unit (40) is configured to store identification boundary data for distinguishing an abnormality of the sensor from a normal state of the sensor;
the electronic control unit (30) is configured to perform the fast Fourier transform on the signal that is the actual data at a first sampling frequency that is equal to or less than 10 Hz to generate first frequency domain data that is the frequency domain data, and perform the fast Fourier transform on the signal that is the actual data at a second sampling frequency that is greater than 10 Hz to generate second frequency domain data; and
the electronic control unit (30) is configured to quantize data with a frequency greater than half the second sampling frequency among the second frequency domain data into a predetermined number of frequency bands to generate second target input data, and determine whether the second target input data is data corresponding to an abnormality or data corresponding to a normal state, based on the identification boundary data.

8. The information processing system according to claim 7, **characterized in that** the electronic control unit (30) is configured to i) perform the fast Fourier transform on the signal at the second sampling frequency that is greater than 10 Hz to generate the second frequency domain data, ii) quantize the data with the frequency greater than half the second sampling frequency among the second frequency domain data into the predetermined number of frequency bands to generate abnormality identification data, iii) perform machine learning using the abnormality identification data as input data, to generate the identification boundary data, and iv) to store the identification boundary data in the storage unit (40).
